# EUROPEAN PATENT APPLICATION

(11) **EP 2 570 475 A1**
(43) Date of publication of application: **20.03.2013**
(21) Application number: 11181111.3
(22) Date of filing: 13.09.2011
(51) Int. Cl.: C11D 3/386

(54) **Detergent composition comprising peptidoglycan-digesting enzyme**

(71) Applicant: The Procter & Gamble Company, Cincinnati, OH 45202 (US)
(72) Inventor: Meek, Michelle, Gateshead, Tyne and Wear NE9 5BD (GB); Bewick, Lindsay Suzanne, Northumberland, NE42 6PP (GB); Souter, Philip Frank, Northumberland, NE65 8UP (GB)
(74) Representative: Howard, Phillip Jan

(57) **Abstract**

A detergent composition containing: (a) peptidoglycan-digesting enzyme having an amino acid sequence identity of at least 80% to the amino acid SEQ ID NO: 1, 2 or 3; (b) detersive surfactant; and a detergent ingredient selected from the group consisting of: (c) perfume microcapsule; (d) a builder system that is: (i) substantially free of phosphate builder; (ii) substantially free of zeolite builder; and (iii) optionally, substantially free of silicate salt; (e) protease system comprising: (i) protease; and (ii) stabiliser system selected from the group consisting of: borate, polyol, 4-formylphenylboronic acid, encapsulating material, and any mixture thereof; and (f) any mixture thereof.

## Description

### FIELD OF THE INVENTION

The present invention relates to solid detergent compositions comprising peptidoglycan-digesting enzyme. The compositions exhibit an excellent freshness profile.

### BACKGROUND OF THE INVENTION

Consumers of laundry detergent products desire not only clean clothes from their laundry products, but also fresh clothes. Consumers especially desire excellent freshness the first time they wear a garment after it has been laundered. There remains a need to improve the freshness profile of laundry detergent compositions. Typically, detergent manufactures have developed sophisticated perfume technologies to achieve this improved freshness. However, the development of a perfume technology to improve freshness can limit the breadth of perfume palate available to the detergent manufacturer. For example, when a perfume system is designed to deliver improved freshness, the product fragrance is constrained by the choice of compatible perfume raw materials; i.e. the flexibility of the detergent manufacturer to deliver improved freshness profile and broad product fragrances is constrained.

The Inventors have found that the freshness and/or cleaning profile can be improved by the use of a specific enzyme system. The Inventors have found that peptidoglycan-digesting enzyme when incorporated into detergent compositions in combination with other specific technologies improves the freshness and/or cleaning profile of the detergent composition. The Inventors have designed an enzyme system to deliver improved freshness profile, which means that a wide variety of perfumes can now be incorporated into the laundry detergent, which in turn enables the detergent manufacturer to choose the product fragrance from broad perfume palate.

### SUMMARY OF THE INVENTION

The present invention provides a detergent composition comprising: (a) peptidoglycan-digesting enzyme having an amino acid sequence identity of at least 80% to the amino acid SEQ ID NO: 1, 2 or 3; (b) detersive surfactant; and a detergent ingredient selected from the group consisting of: (c) perfume microcapsule; (d) a builder system that is: (i) substantially free of phosphate builder; (ii) substantially free of zeolite builder; and (iii) optionally, substantially free of silicate salt; (e) protease system comprising: (i) protease; and (ii) stabiliser system selected from the group consisting of: borate, polyol, 4-formylphenylboronic acid, encapsulating material, and any mixture thereof; and (f) any mixture thereof.

### DETAILED DESCRIPTION OF THE INVENTION

### Detergent composition:

The composition can be any form, for example a liquid or gel form, a solid form, or any combination thereof. The composition may be in a unit dose form, for example a pouch and/or tablet. Suitable pouches include a multi-compartment pouch, especially a multi-compartment pouch that comprises one or more compartments that comprise a solid composition, and one or more compartments that comprise a liquid composition.

The composition is typically a fully finished laundry detergent composition; the composition is not just a component of a laundry detergent composition that can be incorporated into a laundry detergent composition. That said, it is within the scope of the present invention for an additional rinse additive composition (e.g. fabric conditioner or enhancer), or a main wash additive composition (e.g. bleach additive) to also be used in combination with the laundry detergent composition. However, it may be preferred that no bleach additive composition be used in combination with the laundry detergent composition.

The detergent composition comprises: (a) peptidoglycan-digesting enzyme having an amino acid sequence identity of at least 80% to the amino acid SEQ ID NO: 1, 2 or 3; (b) detersive surfactant; and a detergent ingredient selected from the group consisting of: (c) perfume microcapsule; (d) a builder system that is: (i) substantially free of phosphate builder; (ii) substantially free of zeolite builder; and (iii) optionally, substantially free of silicate salt; (e) protease system comprising: (i) protease; and (ii) stabiliser system selected from the group consisting of: borate, polyol, 4-formylphenylboronic acid, encapsulating material, and any mixture thereof; and (f) any mixture thereof.

Typically, the composition is a fully formulated laundry detergent composition, not a portion thereof such as a spray-dried or agglomerated particle that only forms part of the laundry detergent composition. However, it is within the scope of the present invention for an additional rinse additive composition (e.g. fabric conditioner or enhancer), or a main wash additive composition (e.g. bleach additive) to also be used in combination with the laundry detergent composition during the method of the present invention. Although, it may be preferred for no bleach additive composition is used in combination with the laundry detergent composition during the method of the present invention.

Typically, the composition comprises a plurality of chemically different particles, such as spray-dried base detergent particles and/or agglomerated base detergent particles and/or extruded base detergent particles, in combination with one or more, typically two or more, or three or more, or four or more, or five or more, or six or more, or even ten or more particles selected from: surfactant particles, including surfactant agglomerates, surfactant extrudates, surfactant needles, surfactant noodles, surfactant flakes; polymer particles such as cellulosic polymer particles, polyester particles, polyamine particles, terephthalate polymer particles, polyethylene glycol polymer particles; builder particles, such as sodium carbonate and sodium silicate co-builder particles, phosphate particles, zeolite particles, silicate salt particles, carbonate salt particles; filler particles such as sulphate salt particles; dye transfer inhibitor particles; dye fixative particles; bleach particles, such as percarbonate particles, especially coated percarbonate particles, such as percarbonate coated with carbonate salt, sulphate salt, silicate salt, borosilicate salt, or any combination thereof, perborate particles, bleach catalyst particles such as transition metal bleach catalyst particles, or oxaziridinium-based bleach catalyst particles, pre-formed peracid particles, especially coated pre-formed peracid particles, and co-bleach particles of bleach activator, source of hydrogen peroxide and optionally bleach catalyst; bleach activator particles such as oxybenzene sulphonate bleach activator particles and tetra acetyl ethylene diamine bleach activator particles; chelant particles such as chelant agglomerates; hueing dye particles; brightener particles; enzyme particles such as protease prills, lipase prills, cellulase prills, amylase prills, mannanase prills, pectate lyase prills, xyloglucanase prills, bleaching enzyme prills, cutinase prills and co-prills of any of these enzymes; clay particles such as montmorillonite particles or particles of clay and silicone; flocculant particles such as polyethylene oxide particles; wax particles such as wax agglomerates; perfume particles such as perfume microcapsules, especially melamine formaldehyde-based perfume microcapsules, starch encapsulated perfume accord particles, and pro-perfume particles such as Schiff base reaction product particles; aesthetic particles such as coloured noodles or needles or lamellae particles, and soap rings including coloured soap rings; and any combination thereof.

**Detergent ingredients:** The composition typically comprises detergent ingredients. Suitable detergent ingredients include: detersive surfactants including anionic detersive surfactants, non-ionic detersive surfactants, cationic detersive surfactants, zwitterionic detersive surfactants, amphoteric detersive surfactants, and any combination thereof; polymers including carboxylate polymers, polyethylene glycol polymers, polyester soil release polymers such as terephthalate polymers, amine polymers, cellulosic polymers, dye transfer inhibition polymers, dye lock polymers such as a condensation oligomer produced by condensation of imidazole and epichlorhydrin, optionally in ratio of 1:4:1, hexamethylenediamine derivative polymers, and any combination thereof; builders including zeolites, phosphates, citrate, and any combination thereof; buffers and alkalinity sources including carbonate salts and/or silicate salts; fillers including sulphate salts and bio-filler materials; bleach including bleach activators, sources of available oxygen, pre-formed peracids, bleach catalysts, reducing bleach, and any combination thereof; chelants; photobleach; hueing agents; brighteners; enzymes including proteases, amylases, cellulases, lipases, xylogucanases, pectate lyases, mannanases, bleaching enzymes, cutinases, and any combination thereof; enzyme stabilizers; fabric softeners including clay, silicones, quaternary ammonium fabric-softening agents, and any combination thereof; flocculants such as polyethylene oxide; perfume including starch encapsulated perfume accords, perfume microcapsules, perfume loaded zeolites, schif base reaction products of ketone perfume raw materials and polyamines, blooming perfumes, and any combination thereof; aesthetics including soap rings, lamellar aesthetic particles, geltin beads, carbonate and/or sulphate salt speckles, coloured clay, and any combination thereof: and any combination thereof.

**Peptidoglycan digesting enzyme.** Suitable peptidoglycan digesting enzymes are selected from the group comprising chitinases, lysozymes, muramidases and any mixture thereof.

In a preferred embodiment, the peptidoglycan digesting enzyme is a peptidoglycan digesting enzyme which has at least 60%, preferably at least 65%, preferably at least 70%, preferably at least 75% preferably at least 80%, preferably at least 85%, preferably at least 90%, especially preferably at least 95%, preferably at least 99% identity to the polypeptide of SEQ ID NO: 1, 2 or 3.

In another embodiment of the invention, the peptidoglycan digesting enzymes has one of the amino acid sequences shown in SEQ ID NO: 1, 2 or 3.

**Detersive surfactant:** The composition typically comprises detersive surfactant. Suitable detersive surfactants include anionic detersive surfactants, non-ionic detersive surfactant, cationic detersive surfactants, zwitterionic detersive surfactants, amphoteric detersive surfactants, and any combination thereof.

**Anionic detersive surfactant:** Suitable anionic detersive surfactants include sulphate and sulphonate detersive surfactants.

Suitable sulphonate detersive surfactants include alkyl benzene sulphonate, such as C₁₀₋₁₃ alkyl benzene sulphonate. Suitable alkyl benzene sulphonate (LAS) is obtainable, or even obtained, by sulphonating commercially available linear alkyl benzene (LAB); suitable LAB includes low 2-phenyl LAB, such as those supplied by Sasol under the tradename Isochem® or those supplied by Petresa under the tradename Petrelab®, other suitable LAB include high 2-phenyl LAB, such as those supplied by Sasol under the tradename Hyblene®. Another suitable anionic detersive surfactant is alkyl benzene sulphonate that is obtained by DETAL catalyzed process, although other synthesis routes, such as HF, may also be suitable.

Suitable sulphate detersive surfactants include alkyl sulphate, such as C₈₋₁₈ alkyl sulphate, or predominantly C₁₂ alkyl sulphate. The alkyl sulphate may be derived from natural sources, such as coco and/or tallow. Alternative, the alkyl sulphate may be derived from synthetic sources such as C₁₂₋₁₅ alkyl sulphate.

Another suitable sulphate detersive surfactant is alkyl alkoxylated sulphate, such as alkyl ethoxylated sulphate, or a C₈₋₁₈ alkyl alkoxylated sulphate, or a C₈₋₁₈ alkyl ethoxylated sulphate. The alkyl alkoxylated sulphate may have an average degree of alkoxylation of from 0.5 to 20, or from 0.5 to 10. The alkyl alkoxylated sulphate may be a C₈₋₁₈ alkyl ethoxylated sulphate, typically having an average degree of ethoxylation of from 0.5 to 10, or from 0.5 to 7, or from 0.5 to 5 or from 0.5 to 3.

The alkyl sulphate, alkyl alkoxylated sulphate and alkyl benzene sulphonates may be linear or branched, substituted or un-substituted.

The anionic detersive surfactant may be a mid-chain branched anionic detersive surfactant, such as a mid-chain branched alkyl sulphate and/or a mid-chain branched alkyl benzene sulphonate. The mid-chain branches are typically C₁₋₄ alkyl groups, such as methyl and/or ethyl groups.

Another suitable anionic detersive surfactant is alkyl ethoxy carboxylate.

The anionic detersive surfactants are typically present in their salt form, typically being complexed with a suitable cation. Suitable counter-ions include Na⁺ and K⁺, substituted ammonium such as C₁-C₆ alkanolammnonium such as mono-ethanolamine (MEA) tri-ethanolamine (TEA), di-ethanolamine (DEA), and any mixture thereof.

**Non-ionic detersive surfactant:** Suitable non-ionic detersive surfactants are selected from the group consisting of: C₈-C₁₈ alkyl ethoxylates, such as, NEODOL® non-ionic surfactants from Shell; C₆-C₁₂ alkyl phenol alkoxylates wherein optionally the alkoxylate units are ethyleneoxy units, propyleneoxy units or a mixture thereof; C₁₂-C₁₈ alcohol and C₆-C₁₂ alkyl phenol condensates with ethylene oxide/propylene oxide block polymers such as Pluronic® from BASF; C₁₄-C₂₂ mid-chain branched alcohols; C₁₄-C₂₂ mid-chain branched alkyl alkoxylates, typically having an average degree of alkoxylation of from 1 to 30; alkylpolysaccharides, such as alkylpolyglycosides; polyhydroxy fatty acid amides; ether capped poly(oxyalkylated) alcohol surfactants; and mixtures thereof.

Suitable non-ionic detersive surfactants are alkyl polyglucoside and/or an alkyl alkoxylated alcohol.

Suitable non-ionic detersive surfactants include alkyl alkoxylated alcohols, such as C₈₋₁₈ alkyl alkoxylated alcohol, or a C₈₋₁₈ alkyl ethoxylated alcohol. The alkyl alkoxylated alcohol may have an average degree of alkoxylation of from 0.5 to 50, or from 1 to 30, or from 1 to 20, or from 1 to 10. The alkyl alkoxylated alcohol may be a C₈₋₁₈ alkyl ethoxylated alcohol, typically having an average degree of ethoxylation of from 1 to 10, or from 1 to 7, or from 1 to 5, or from 3 to 7. The alkyl alkoxylated alcohol can be linear or branched, and substituted or un-substituted.

Suitable nonionic detersive surfactants include secondary alcohol-based detersive surfactants having the formula: 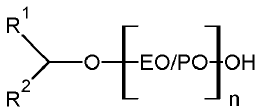
wherein R¹ = linear or branched, substituted or unsubstituted, saturated or unsaturated C₂₋₈ alkyl;
wherein R² = linear or branched, substituted or unsubstituted, saturated or unsaturated C₂₋₈ alkyl,
wherein the total number of carbon atoms present in R¹ + R² moieties is in the range of from 7 to 13;
wherein EO/PO are alkoxy moieties selected from ethoxy, propoxy, or mixtures thereof, optionally the EO/PO alkoxyl moieties are in random or block configuration;
wherein n is the average degree of alkoxylation and is in the range of from 4 to 10.

Other suitable non-ionic detersive surfactants include EO/PO block co-polymer surfactants, such as the Plurafac^{®} series of surfactants available from BASF, and sugar-derived surfactants such as alkyl N-methyl glucose amide.

**Cationic detersive surfactant:** Suitable cationic detersive surfactants include alkyl pyridinium compounds, alkyl quaternary ammonium compounds, alkyl quaternary phosphonium compounds, alkyl ternary sulphonium compounds, and mixtures thereof.

Suitable cationic detersive surfactants are quaternary ammonium compounds having the general formula:

(R)(R₁)(R₂)(R₃)N⁺ X⁻

wherein, R is a linear or branched, substituted or unsubstituted C₆₋₁₈ alkyl or alkenyl moiety, R₁ and R₂ are independently selected from methyl or ethyl moieties, R₃ is a hydroxyl, hydroxymethyl or a hydroxyethyl moiety, X is an anion which provides charge neutrality, suitable anions include: halides, such as chloride; sulphate; and sulphonate. Suitable cationic detersive surfactants are mono-C₆₋₁₈ alkyl mono-hydroxyethyl di-methyl quaternary ammonium chlorides. Suitable cationic detersive surfactants are mono-C₈₋₁₈ alkyl mono-hydroxyethyl di-methyl quaternary ammonium chloride, mono-C₁₀₋₁₂ alkyl mono-hydroxyethyl di-methyl quaternary ammonium chloride and mono-C₁₀ alkyl mono-hydroxyethyl di-methyl quaternary ammonium chloride.

**Zwitterionic and/or amphoteric detersive surfactant:** Suitable zwitterionic and/or amphoteric detersive surfactants include amine oxide such as dodecyldimethylamine N-oxide, alkanolamine sulphobetaines, coco-amidopropyl betaines, HN⁺-R-CO₂⁻ based surfactants, wherein R can be any bridging group, such as alkyl, alkoxy, aryl or amino acids.

**Polymer:** Suitable polymers include carboxylate polymers, polyethylene glycol polymers, polyester soil release polymers such as terephthalate polymers, amine polymers, cellulosic polymers, dye transfer inhibition polymers, dye lock polymers such as a condensation oligomer produced by condensation of imidazole and epichlorhydrin, optionally in ratio of 1:4:1, hexamethylenediamine derivative polymers, and any combination thereof.

**Carboxylate polymer:** Suitable carboxylate polymers include maleate/acrylate random copolymer or polyacrylate homopolymer. The carboxylate polymer may be a polyacrylate homopolymer having a molecular weight of from 4,000 Da to 9,000 Da, or from 6,000 Da to 9,000 Da. Other suitable carboxylate polymers are co-polymers of maleic acid and acrylic acid, and may have a molecular weight in the range of from 4,000 Da to 90,000 Da.

**Polyethylene glycol polymer:** Suitable polyethylene glycol polymers include random graft co-polymers comprising: (i) hydrophilic backbone comprising polyethylene glycol; and (ii) hydrophobic side chain(s) selected from the group consisting of: C₄-C₂₅ alkyl group, polypropylene, polybutylene, vinyl ester of a saturated C₁-C₆ mono-carboxylic acid, C₁₋C₆ alkyl ester of acrylic or methacrylic acid, and mixtures thereof. Suitable polyethylene glycol polymers have a polyethylene glycol backbone with random grafted polyvinyl acetate side chains. The average molecular weight of the polyethylene glycol backbone can be in the range of from 2,000 Da to 20,000 Da, or from 4,000 Da to 8,000 Da. The molecular weight ratio of the polyethylene glycol backbone to the polyvinyl acetate side chains can be in the range of from 1:1 to 1:5, or from 1:1.2 to 1:2. The average number of graft sites per ethylene oxide units can be less than 1, or less than 0.8, the average number of graft sites per ethylene oxide units can be in the range of from 0.5 to 0.9, or the average number of graft sites per ethylene oxide units can be in the range of from 0.1 to 0.5, or from 0.2 to 0.4. A suitable polyethylene glycol polymer is Sokalan HP22.

**Polyester soil release polymers:** Suitable polyester soil release polymers have a structure as defined by one of the following structures (I), (II) or (III):
(I) -[(OCHR¹-CHR²)ₐ-O-OC-Ar-CO-]_{d}
(II) -[(OCHR₃-CHR⁴)_{b}-O-OC-sAr-CO-]ₑ
(III) -[(OCHR⁵-CHR⁶)_{c}-OR⁷]_{f}
wherein:
a, b and c are from 1 to 200;
d, e and f are from 1 to 50;
Ar is a 1,4-substituted phenylene;
sAr is 1,3-substituted phenylene substituted in position 5 with SO₃Me;
Me is Li, K, Mg/2, Ca/2, Al/3, ammonium, mono-, di-, tri-, or tetraalkylammonium wherein the alkyl groups are C₁-C₁₈ alkyl or C₂-C₁₀ hydroxyalkyl, or any mixture thereof;
R¹, R², R³, R⁴, R⁵ and R⁶ are independently selected from H or C₁-C₁₈ n- or iso-alkyl; and
R⁷ is a linear or branched C₁-C₁₈ alkyl, or a linear or branched C₂-C₃₀ alkenyl, or a cycloalkyl group with 5 to 9 carbon atoms, or a C₈-C₃₀ aryl group, or a C₆-C₃₀ arylalkyl group. Suitable polyester soil release polymers are terephthalate polymers having the structure of formula (I) or (II) above.

Suitable polyester soil release polymers include the Repel-o-tex series of polymers such as Repel-o-tex SF2 (Rhodia) and/or the Texcare series of polymers such as Texcare SRA300 (Clariant).

**Amine polymer:** Suitable amine polymers include polyethylene imine polymers, such as alkoxylated polyalkyleneimines, optionally comprising a polyethylene and/or polypropylene oxide block.

**Cellulosic polymer:** The composition can comprise cellulosic polymers, such as polymers selected from alkyl cellulose, alkyl alkoxyalkyl cellulose, carboxyalkyl cellulose, alkyl carboxyalkyl, and any combination thereof. Suitable cellulosic polymers are selected from carboxymethyl cellulose, methyl cellulose, methyl hydroxyethyl cellulose, methyl carboxymethyl cellulose, and mixtures thereof. The carboxymethyl cellulose can have a degree of carboxymethyl substitution from 0.5 to 0.9 and a molecular weight from 100,000 Da to 300,000 Da. Another suitable cellulosic polymer is hydrophobically modified carboxymethyl cellulose, such as Finnfix SH-1 (CP Kelco).

Other suitable cellulosic polymers may have a degree of substitution (DS) of from 0.01 to 0.99 and a degree of blockiness (DB) such that either DS+DB is of at least 1.00 or DB+2DS-DS² is at least 1.20. The substituted cellulosic polymer can have a degree of substitution (DS) of at least 0.55. The substituted cellulosic polymer can have a degree of blockiness (DB) of at least 0.35. The substituted cellulosic polymer can have a DS + DB, of from 1.05 to 2.00. A suitable substituted cellulosic polymer is carboxymethylcellulose.

Another suitable cellulosic polymer is cationically modified hydroxyethyl cellulose.

**Dye transfer inhibitor polymer:** Suitable dye transfer inhibitor (DTI) polymers include polyvinyl pyrrolidone (PVP), vinyl co-polymers of pyrrolidone and imidazoline (PVPVI), polyvinyl N-oxide (PVNO), and any mixture thereof.

**Hexamethylenediamine derivative polymers:** Suitable polymers includehexamethylenediamine derivative polymers, typically having the formula:

R₂(CH₃)N⁺(CH₂)6N⁺(CH₃)R₂. 2X⁻

wherein X⁻ is a suitable counter-ion, for example chloride, and R is a poly(ethylene glycol) chain having an average degree of ethoxylation of from 20 to 30. Optionally, the poly(ethylene glycol) chains may be independently capped with sulphate and/or sulphonate groups, typically with the charge being balanced by reducing the number of X⁻ counter-ions, or (in cases where the average degree of sulphation per molecule is greater than two), introduction of Y⁺ counter-ions, for example sodium cations.

**Builder:** Suitable builders include zeolites, phosphates, citrates, and any combination thereof.

**Zeolite builder:** The composition typically comprises from 0wt% to 10wt%, zeolite builder, or to 8wt%, or to 6wt%, or to 4wt%, or to 3wt%, or to 2wt%, or even to 1wt% zeolite builder. The composition may even be substantially free of zeolite builder; substantially free means "no deliberately added". Typical zeolite builders include zeolite A, zeolite P, zeolite MAP, zeolite X and zeolite Y.

**Phosphate builder:** The composition typically comprises from 0wt% to 10wt% phosphate builder, or to 8wt%, or to 6wt%, or to 4wt%, or to 3wt%, or to 2wt%, or even to 1wt% phosphate builder. The composition may even be substantially free of phosphate builder; substantially free means "no deliberately added". A typical phosphate builder is sodium tri-polyphosphate (STPP).

**Citrate:** A suitable citrate is sodium citrate. However, citric acid may also be incorporated into the composition, which can form citrate in the wash liquor.

**Buffer and alkalinity source:** Suitable buffers and alkalinity sources include carbonate salts and/or silicate salts and/or double salts such as burkeitte.

**Carbonate salt:** A suitable carbonate salt is sodium carbonate and/or sodium bicarbonate. The composition may comprise bicarbonate salt. It may be suitable for the composition to comprise low levels of carbonate salt, for example, it may be suitable for the composition to comprise from 0wt% to 10wt% carbonate salt, or to 8wt%, or to 6wt%, or to 4wt%, or to 3wt%, or to 2wt%, or even to 1wt% carbonate salt. The composition may even be substantially free of carbonate salt; substantially free means "no deliberately added".

The carbonate salt may have a weight average mean particle size of from 100 to 500 micrometers. Alternatively, the carbonate salt may have a weight average mean particle size of from 10 to 25 micrometers.

**Silicate salt:** The composition may comprise from 0wt% to 20wt% silicate salt, or to 15wt%, or to 10wt%, or to 5wt%, or to 4wt%, or even to 2wt%, and may comprise from above 0wt%, or from 0.5wt%, or even from 1wt% silicate salt. The silicate can be crystalline or amorphous. Suitable crystalline silicates include crystalline layered silicate, such as SKS-6. Other suitable silicates include 1.6R silicate and/or 2.0R silicate. A suitable silicate salt is sodium silicate. Another suitable silicate salt is sodium metasilicate.

**Filler:** The composition may comprise from 0wt% to 70% filler. Suitable fillers include sulphate salts and/or bio-filler materials.

**Sulphate salt:** A suitable sulphate salt is sodium sulphate. The sulphate salt may have a weight average mean particle size of from 100 to 500 micrometers, alternatively, the sulphate salt may have a weight average mean particle size of from 10 to 45 micrometers.

**Bio-filler material:** A suitable bio-filler material is alkali and/or bleach treated agricultural waste.

**Bleach:** The composition may comprise bleach. Alternatively, the composition may be substantially free of bleach; substantially free means "no deliberately added". Suitable bleach includes bleach activators, sources of available oxygen, pre-formed peracids, bleach catalysts, reducing bleach, and any combination thereof. If present, the bleach, or any component thereof, for example the pre-formed peracid, may be coated, such as encapsulated, or clathrated, such as with urea or cyclodextrin.

**Bleach activator:** Suitable bleach activators include: tetraacetylethylenediamine (TAED); oxybenzene sulphonates such as nonanoyl oxybenzene sulphonate (NOBS), caprylamidononanoyl oxybenzene sulphonate (NACA-OBS), 3,5,5-trimethyl hexanoyloxybenzene sulphonate (Iso-NOBS), dodecyl oxybenzene sulphonate (LOBS), and any mixture thereof; caprolactams; pentaacetate glucose (PAG); nitrile quaternary ammonium; imide bleach activators, such as N-nonanoyl-N-methyl acetamide; and any mixture thereof.

**Source of available oxygen:** A suitable source of available oxygen (AvOx) is a source of hydrogen peroxide, such as percarbonate salts and/or perborate salts, such as sodium percarbonate. The source of peroxygen may be at least partially coated, or even completely coated, by a coating ingredient such as a carbonate salt, a sulphate salt, a silicate salt, borosilicate, or any mixture thereof, including mixed salts thereof. Suitable percarbonate salts can be prepared by a fluid bed process or by a crystallization process. Suitable perborate salts include sodium perborate mono-hydrate (PB1), sodium perborate tetra-hydrate (PB4), and anhydrous sodium perborate which is also known as fizzing sodium perborate. Other suitable sources of AvOx include persulphate, such as oxone. Another suitable source of AvOx is hydrogen peroxide.

**Pre-formed peracid:** A suitable pre-formed peracid is N,N-pthaloylamino peroxycaproic acid (PAP).

**Bleach catalyst:** Suitable bleach catalysts include oxaziridinium-based bleach catalysts, transition metal bleach catalysts and bleaching enzymes.

**Oxaziridinium-based bleach catalyst:** A suitable oxaziridinium-based bleach catalyst has the formula: 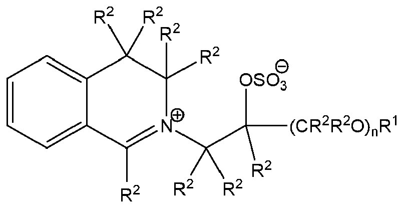 wherein: R¹ is selected from the group consisting of: H, a branched alkyl group containing from 3 to 24 carbons, and a linear alkyl group containing from 1 to 24 carbons; R¹ can be a branched alkyl group comprising from 6 to 18 carbons, or a linear alkyl group comprising from 5 to 18 carbons, R¹ can be selected from the group consisting of: 2-propylheptyl, 2-butyloctyl, 2-pentylnonyl, 2-hexyldecyl, n-hexyl, n-octyl, n-decyl, n-dodecyl, n-tetradecyl, n-hexadecyl, n-octadecyl, iso-nonyl, iso-decyl, iso-tridecyl and iso-pentadecyl; R² is independently selected from the group consisting of: H, a branched alkyl group comprising from 3 to 12 carbons, and a linear alkyl group comprising from 1 to 12 carbons; optionally R² is independently selected from H and methyl groups; and n is an integer from 0 to 1.

**Transition metal bleach catalyst:** The composition may include transition metal bleach catalyst, typically comprising copper, iron, titanium, ruthenium, tungsten, molybdenum, and/or manganese cations. Suitable transition metal bleach catalysts are manganese-based transition metal bleach catalysts.

**Reducing bleach:** The composition may comprise a reducing bleach. However, the composition may be substantially free of reducing bleach; substantially free means "no deliberately added". Suitable reducing bleach include sodium sulphite and/or thiourea dioxide (TDO).

**Co-bleach particle:** The composition may comprise a co-bleach particle. Typically, the co-bleach particle comprises a bleach activator and a source of peroxide. It may be highly suitable for a large amount of bleach activator relative to the source of hydrogen peroxide to be present in the co-bleach particle. The weight ratio of bleach activator to source of hydrogen peroxide present in the co-bleach particle can be at least 0.3:1, or at least 0.6:1, or at least 0.7:1, or at least 0.8:1, or at least 0.9:1, or at least 1.0:1.0, or even at least 1.2:1 or higher.

The co-bleach particle can comprise: (i) bleach activator, such as TAED; and (ii) a source of hydrogen peroxide, such as sodium percarbonate. The bleach activator may at least partially, or even completely, enclose the source of hydrogen peroxide.

The co-bleach particle may comprise a binder. Suitable binders are carboxylate polymers such as polyacrylate polymers, and/or surfactants including non-ionic detersive surfactants and/or anionic detersive surfactants such as linear C₁₁-C₁₃ alkyl benzene sulphonate.

The co-bleach particle may comprise bleach catalyst, such as an oxaziridium-based bleach catalyst.

**Chelant:** Suitable chelants are selected from: diethylene triamine pentaacetate, diethylene triamine penta(methyl phosphonic acid), ethylene diamine-N'N'-disuccinic acid, ethylene diamine tetraacetate, ethylene diamine tetra(methylene phosphonic acid), hydroxyethane di(methylene phosphonic acid), and any combination thereof. A suitable chelant is ethylene diamine-N'N'-disuccinic acid (EDDS) and/or hydroxyethane diphosphonic acid (HEDP). The laundry detergent composition may comprise ethylene diamine-N'N'- disuccinic acid or salt thereof. The ethylene diamine-N'N'-disuccinic acid may be in S,S enantiomeric form. The composition may comprise 4,5-dihydroxy-m-benzenedisulfonic acid disodium salt. Suitable chelants may also be calcium crystal growth inhibitors.

**Calcium carbonate crystal growth inhibitor:** The composition may comprise a calcium carbonate crystal growth inhibitor, such as one selected from the group consisting of: 1-hydroxyethanediphosphonic acid (HEDP) and salts thereof; N,N-dicarboxymethyl-2-aminopentane-1,5-dioic acid and salts thereof; 2-phosphonobutane-1,2,4-tricarboxylic acid and salts thereof; and any combination thereof.

**Photobleach:** Suitable photobleaches are zinc and/or aluminium sulphonated phthalocyanines.

**Hueing agent:** The hueing agent (also defined herein as hueing dye) is typically formulated to deposit onto fabrics from the wash liquor so as to improve fabric whiteness perception. The hueing agent is typically blue or violet. It may be suitable that the hueing dye(s) have a peak absorption wavelength of from 550nm to 650nm, or from 570nm to 630nm. The hueing agent may be a combination of dyes which together have the visual effect on the human eye as a single dye having a peak absorption wavelength on polyester of from 550nm to 650nm, or from 570nm to 630nm. This may be provided for example by mixing a red and green-blue dye to yield a blue or violet shade.

Dyes are typically coloured organic molecules which are soluble in aqueous media that contain surfactants. Dyes maybe selected from the classes of basic, acid, hydrophobic, direct and polymeric dyes, and dye-conjugates. Suitable polymeric hueing dyes are commercially available, for example from Milliken, Spartanburg, South Carolina, USA.

Examples of suitable dyes are violet DD, direct violet 7 , direct violet 9 , direct violet 11, direct violet 26, direct violet 31, direct violet 35, direct violet 40, direct violet 41, direct violet 51, direct violet 66, direct violet 99, acid violet 50, acid blue 9, acid violet 17, acid black 1 , acid red 17, acid blue 29, solvent violet 13, disperse violet 27 disperse violet 26, disperse violet 28, disperse violet 63 and disperse violet 77, basic blue 16, basic blue 65, basic blue 66, basic blue 67, basic blue 71, basic blue 159, basic violet 19, basic violet 35, basic violet 38, basic violet 48; basic blue 3 , basic blue 75, basic blue 95, basic blue 122, basic blue 124, basic blue 141, thiazolium dyes, reactive blue 19, reactive blue 163, reactive blue 182, reactive blue 96, Liquitint® Violet CT (Milliken, Spartanburg, USA) and Azo-CM-Cellulose (Megazyme, Bray, Republic of Ireland). Other suitable hueing agents are hueing dye-photobleach conjugates, such as the conjugate of sulphonated zinc phthalocyanine with direct violet 99. A particularly suitable hueing agent is a combination of acid red 52 and acid blue 80, or the combination of direct violet 9 and solvent violet 13.

**Brightener:** Suitable brighteners are stilbenes, such as brightener 15. Other suitable brighteners are hydrophobic brighteners, and brightener 49. The brightener may be in micronized particulate form, having a weight average particle size in the range of from 3 to 30 micrometers, or from 3 micrometers to 20 micrometers, or from 3 to 10 micrometers. The brightener can be alpha or beta crystalline form.

**Enzyme:** Suitable enzymes include proteases, amylases, cellulases, lipases, xylogucanases, pectate lyases, mannanases, bleaching enzymes, cutinases, and mixtures thereof.

For the enzymes, accession numbers and IDs shown in parentheses refer to the entry numbers in the databases Genbank, EMBL and/or Swiss-Prot. For any mutations, standard 1-letter amino acid codes are used with a * representing a deletion. Accession numbers prefixed with DSM refer to micro-organisms deposited at Deutsche Sammlung von Mikroorganismen und Zellkulturen GmbH, Mascheroder Weg 1b, 38124 Brunswick (DSMZ).

**Protease.** The composition may comprise a protease. Suitable proteases include metalloproteases and/or serine proteases, including neutral or alkaline microbial serine proteases, such as subtilisins (EC 3.4.21.62). Suitable proteases include those of animal, vegetable or microbial origin. In one aspect, such suitable protease may be of microbial origin. The suitable proteases include chemically or genetically modified mutants of the aforementioned suitable proteases. In one aspect, the suitable protease may be a serine protease, such as an alkaline microbial protease or/and a trypsin-type protease. Examples of suitable neutral or alkaline proteases include:
(a) subtilisins (EC 3.4.21.62), including those derived from *Bacillus,* such as *Bacillus lentus, Bacillus alkalophilus* (P27963, ELYA_BACAO), *Bacillus subtilis, Bacillus amyloliquefaciens* (P00782, SUBT_BACAM), *Bacillus pumilus (P07518) and Bacillus gibsonii* (DSM14391).
(b) trypsin-type or chymotrypsin-type proteases, such as trypsin (e.g. of porcine or bovine origin), including the Fusarium protease and the chymotrypsin proteases derived from *Cellumonas* (A2RQE2).
(c) metalloproteases, including those derived from *Bacillus amyloliquefaciens* (P06832, NPRE_BACAM).

Suitable proteases include those derived from *Bacillus gibsonii* or *Bacillus Lentus* such as subtilisin 309 (P29600) and/or DSM 5483 (P29599).

Suitable commercially available protease enzymes include: those sold under the trade names Alcalase®, Savinase®, Primase®, Durazym®, Polarzyme®, Kannase®, Liquanase®, Liquanase Ultra®, Savinase Ultra®, Ovozyme®, Neutrase®, Everlase® and Esperase® by Novozymes A/S (Denmark); those sold under the tradename Maxatase®, Maxacal®, Maxapem®, Properase®, Purafect®, Purafect Prime®, Purafect Ox®, FN3®, FN4®, Excellase® and Purafect OXP® by Genencor International; those sold under the tradename Opticlean® and Optimase® by Solvay Enzymes; those available from Henkel/Kemira, namely BLAP (P29599 having the following mutations S99D + S101 R + S103A + V104I + G159S), and variants thereof including BLAP R (BLAP with S3T + V4I + V199M + V205I + L217D), BLAP X (BLAP with S3T + V4I + V205I) and BLAP F49 (BLAP with S3T + V4I + A194P + V199M + V205I + L217D) all from Henkel/Kemira; and KAP (Bacillus alkalophilus subtilisin with mutations A230V + S256G + S259N) from Kao.

**Amylase:** Suitable amylases are alpha-amylases, including those of bacterial or fungal origin. Chemically or genetically modified mutants (variants) are included. A suitable alkaline alpha-amylase is derived from a strain of *Bacillus,* such as *Bacillus licheniformis, Bacillus amyloliquefaciens, Bacillus stearothermophilus, Bacillus subtilis,* or other *Bacillus sp.,* such as *Bacillus sp.* NCIB 12289, NCIB 12512, NCIB 12513, sp 707, DSM 9375, DSM 12368, DSMZ no. 12649, KSM AP1378, KSM K36 or KSM K38. Suitable amylases include:
(a) alpha-amylase derived from *Bacillus licheniformis* (P06278, AMY_BACLI), and variants thereof, especially the variants with substitutions in one or more of the following positions: 15, 23, 105, 106, 124, 128, 133, 154, 156, 181, 188, 190, 197, 202, 208, 209, 243, 264, 304, 305, 391, 408, and 444.
(b) AA560 amylase (CBU30457, HD066534) and variants thereof, especially the variants with one or more substitutions in the following positions: 26, 30, 33, 82, 37, 106, 118, 128, 133, 149, 150, 160, 178, 182, 186, 193, 203, 214, 231, 256, 257, 258, 269, 270, 272, 283, 295, 296, 298, 299, 303, 304, 305, 311, 314, 315, 318, 319, 339, 345, 361, 378, 383, 419, 421, 437, 441, 444, 445, 446, 447, 450, 461, 471, 482, 484, optionally that also contain the deletions of D183* and G184*.
(c) variants exhibiting at least 90% identity with the wild-type enzyme from *Bacillus SP722* (CBU30453, HD066526), especially variants with deletions in the 183 and 184 positions.

Suitable commercially available alpha-amylases are Duramyl®, Liquezyme® Termamyl®, Termamyl Ultra®, Natalase®, Supramyl®, Stainzyme®, Stainzyme Plus®, Fungamyl® and BAN® (Novozymes A/S), Bioamylase® and variants thereof (Biocon India Ltd.), Kemzym® AT 9000 (Biozym Ges. m.b.H, Austria), Rapidase®, Purastar®, Optisize HT Plus®, Enzysize®, Powerase® and Purastar Oxam®, Maxamyl® (Genencor International Inc.) and KAM® (KAO, Japan). Suitable amylases are Natalase®, Stainzyme® and Stainzyme Plus®.

**Cellulase:** The composition may comprise a cellulase. Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium,* e.g., the fungal cellulases produced from *Humicola insolens, Myceliophthora thermophila* and *Fusarium oxysporum.*

Commercially available cellulases include Celluzyme®, and Carezyme® (Novozymes A/S), Clazinase®, and Puradax HA® (Genencor International Inc.), and KAC-500(B)® (Kao Corporation).

The cellulase can include microbial-derived endoglucanases exhibiting endo-beta-1,4-glucanase activity (E.C. 3.2.1.4), including a bacterial polypeptide endogenous to a member of the genus *Bacillus sp. AA349* and mixtures thereof. Suitable endoglucanases are sold under the tradenames Celluclean® and Whitezyme® (Novozymes A/S, Bagsvaerd, Denmark).

The composition may comprise a cleaning cellulase belonging to Glycosyl Hydrolase family 45 having a molecular weight of from 17kDa to 30 kDa, for example the endoglucanases sold under the tradename Biotouch® NCD, DCC and DCL (AB Enzymes, Darmstadt, Germany).

Suitable cellulases may also exhibit xyloglucanase activity, such as Whitezyme®.

**Lipase.** The composition may comprise a lipase. Suitable lipases include those of bacterial or fungal origin. Chemically modified or protein engineered mutants are included. Examples of useful lipases include lipases from *Humicola* (synonym *Thermomyces),* e.g., from *H. lanuginosa* (*T. lanuginosus*), or from *H. insolens,* a Pseudomonas lipase, e.g., from *P. alcaligenes* or *P*. *pseudoalcaligenes, P. cepacia, P. stutzeri, P. fluorescens, Pseudomonas* sp. strain SD 705, *P*. *wisconsinensis, a Bacillus* lipase, e.g., from *B. subtilis, B. stearothermophilus* or *B. pumilus.*

The lipase may be a "first cycle lipase", optionally a variant of the wild-type lipase from *Thermomyces lanuginosus* comprising T231R and N233R mutations. The wild-type sequence is the 269 amino acids (amino acids 23 - 291) of the Swissprot accession number Swiss-Prot 059952 (derived from *Thermomyces lanuginosus* (*Humicola lanuginosa*)). Suitable lipases would include those sold under the tradenames Lipex®, Lipolex® and Lipoclean® by Novozymes, Bagsvaerd, Denmark.

The composition may comprise a variant of *Thermomyces lanuginosa* (059952) lipase having >90% identity with the wild type amino acid and comprising substitution(s) at T231 and/or N233, optionally T231R and/or N233R.

**Xyloglucanase:** Suitable xyloglucanase enzymes may have enzymatic activity towards both xyloglucan and amorphous cellulose substrates. The enzyme may be a glycosyl hydrolase (GH) selected from GH families 5, 12, 44 or 74. The glycosyl hydrolase selected from GH family 44 is particularly suitable. Suitable glycosyl hydrolases from GH family 44 are the XYG1006 glycosyl hydrolase from *Paenibacillus polyxyma* (ATCC 832) and variants thereof.

**Pectate lyase:** Suitable pectate lyases are either wild-types or variants of Bacillus-derived pectate lyases (CAF05441, AAU25568) sold under the tradenames Pectawash®, Pectaway® and X-Pect® (from Novozymes A/S, Bagsvaerd, Denmark).

**Mannanase:** Suitable mannanases are sold under the tradenames Mannaway® (from Novozymes A/S, Bagsvaerd, Denmark), and Purabrite® (Genencor International Inc., Palo Alto, California).

**Bleaching enzyme:** Suitable bleach enzymes include oxidoreductases, for example oxidases such as glucose, choline or carbohydrate oxidases, oxygenases, catalases, peroxidases, like halo-, chloro-, bromo-, lignin-, glucose- or manganese-peroxidases, dioxygenases or laccases (phenoloxidases, polyphenoloxidases). Suitable commercial products are sold under the Guardzyme® and Denilite® ranges from Novozymes. It may be advantageous for additional organic compounds, especially aromatic compounds, to be incorporated with the bleaching enzyme; these compounds interact with the bleaching enzyme to enhance the activity of the oxidoreductase (enhancer) or to facilitate the electron flow (mediator) between the oxidizing enzyme and the stain typically over strongly different redox potentials.

Other suitable bleaching enzymes include perhydrolases, which catalyse the formation of peracids from an ester substrate and peroxygen source. Suitable perhydrolases include variants of the Mycobacterium smegmatis perhydrolase, variants of so-called CE-7 perhydrolases, and variants of wild-type subtilisin Carlsberg possessing perhydrolase activity.

**Cutinase:** Suitable cutinases are defined by E.C. Class 3.1.1.73, optionally displaying at least 90%, or 95%, or most optionally at least 98% identity with a wild-type derived from one of *Fusarium solani, Pseudomonas Mendocina* or *Humicola Insolens.*

**Identity.** The relativity between two amino acid sequences is described by the parameter "identity". For purposes of the present invention, the alignment of two amino acid sequences is determined by using the Needle program from the EMBOSS package (http://emboss.org) version 2.8.0. The Needle program implements the global alignment algorithm described in Needleman, S. B. and Wunsch, C. D. (1970) J. Mol. Biol. 48, 443-453. The substitution matrix used is BLOSUM62, gap opening penalty is 10, and gap extension penalty is 0.5.

**Enzyme stabilizer.** The composition may comprise an enzyme stabilizer. This may be preferred when the composition is in liquid form, although enzyme stabilizers may also be used when the composition is in solid form. Suitable enzyme stabilizers include polyols such as propylene glycol or glycerol, sugar or sugar alcohol, lactic acid, reversible protease inhibitor, boric acid, or a boric acid derivative, e.g., an aromatic borate ester, or a phenyl boronic acid derivative such as 4-formylphenyl boronic acid. It may be preferred for the composition to comprise a nil-boron enzyme stabilizer, preferably selected from polyols such as propylene glycol or glycerol, sugar or sugar alcohol. It may even be preferred for the composition to be substantially free of boron. By substantially free it is typically meant: "comprises no deliberately added". Free of boron also typically includes being free of sources of boron such as borax.

**Fabric-softener:** Suitable fabric-softening agents include clay, silicone and/or quaternary ammonium compounds. Suitable clays include montmorillonite clay, hectorite clay and/or laponite clay. A suitable clay is montmorillonite clay. Suitable silicones include amino-silicones and/or polydimethylsiloxane (PDMS). A suitable fabric softener is a particle comprising clay and silicone, such as a particle comprising montmorillonite clay and PDMS.

**Flocculant:** Suitable flocculants include polyethylene oxide; for example having an average molecular weight of from 300,000 Da to 900,000 Da.

**Suds suppressor:** Suitable suds suppressors include silicone and/or fatty acid such as stearic acid.

**Perfume:** Suitable perfumes include perfume microcapsules, polymer assisted perfume delivery systems including Schiff base perfume/polymer complexes, starch-encapsulated perfume accords, perfume-loaded zeolites, blooming perfume accords, and any combination thereof. A suitable perfume microcapsule is melamine formaldehyde based, typically comprising perfume that is encapsulated by a shell comprising melamine formaldehyde. It may be highly suitable for such perfume microcapsules to comprise cationic and/or cationic precursor material in the shell, such as polyvinyl formamide (PVF) and/or cationically modified hydroxyethyl cellulose (catHEC).

**Aesthetic:** Suitable aesthetic particles include soap rings, lamellar aesthetic particles, geltin beads, carbonate and/or sulphate salt speckles, coloured clay particles, and any combination thereof.

**Calcium and Magnesium cations.** Preferably, the composition comprises from at least 0.2wt% to 5wt% calcium and/or magnesium cations.

**Visual signaling ingredients.** Suitable visual signaling ingredients include any reflective and/or refractive material, preferably mica.

**Anti-foam.** The detergent compositions herein comprise from about 0.001wt% to about 4.0wt% anti-foam selected from silicone anti-foam compounds; anti-foam compounds of silicone oils and hydrophobic particles; and mixtures thereof. In one embodiment, the compositions herein comprise from about 0.01wt% to about 2.0wt%, alternatively from 0.05wt% to about 1.0wt% silicone anti-foam (percentages by active amount not including any carrier).

In one embodiment, the anti-foam is selected from: organomodified silicone polymers with aryl or alkylaryl substituents combined with silicone resin and modified silica; M/Q resins; and mixtures thereof.

**Fatty acid.** The composition comprises from 0wt% to 10wt%, preferably from 0wt% to 5wt%, preferably from 0.1wt% to 5wt%, preferably from 0.5wt% to 3wt% saturated or unsaturated fatty acid, preferably saturated or unsaturated C₁₂-C₂₄ fatty acid; highly preferred are saturated C₁₂-C₁₈ fatty acid.

**Structurant/thickener.** Structured liquids can either be internally structured, whereby the structure is formed by primary ingredients (e.g. surfactant material) and/or externally structured by providing a three dimensional matrix structure using secondary ingredients (e.g. polymers, clay and/or silicate material).

The composition may comprise a structurant, preferably from 0.01wt% to 5wt%, from 0.1wt% to 2.0wt% structurant. The structurant is typically selected from the group consisting of diglycerides and triglycerides, ethylene glycol distearate, microcrystalline cellulose, cellulose-based materials, microfiber cellulose, biopolymers, xanthan gum, gellan gum, and mixtures thereof. A suitable structurant includes hydrogenated castor oil, and non-ethoxylated derivatives thereof. It may be preferred for the composition to substantially free of lipase, by substantially free it is typically meant: "comprises no deliberately added". This is especially preferred when the composition comprises hydrogenated castor oil, and non-ethoxylated derivatives thereof. A suitable structurant is US6855680, such structurants have a thread-like structuring system having a range of aspect ratios.

Ethylene glycol distearate can also be used as a visual signaling ingredient.

**Fatty alcohol gel network.** It may be preferred for the composition to comprise a first wash lipase, especially preferably in combination with a gel network, such as a fatty alcohol gel network. C₈-C₁₂ fatty alcohol, such as dodecanol, fatty alcohol gel networks are particularly suitable. Alternatively, gum gel networks can also be used.

**Solvent.** The composition preferably comprises solvent. Preferred solvents include alcohols and/or glycols, preferably methanol, ethanol and/or propylene glycol. Preferably, the composition comprises no or minimal amounts of methanol and ethanol and instead comprises relatively high amounts of propylene glycol, for improved enzyme stability. Preferably, the composition comprises propylene glycol.

Suitable solvents include C₄-C₁₄ ethers and diethers, glycols, alkoxylated glycols, C₆-C₁₆ glycol ethers, alkoxylated aromatic alcohols, aromatic alcohols, aliphatic branched alcohols, alkoxylated aliphatic branched alcohols, alkoxylated linear C₁-C₅ alcohols, linear C₁-C₅ alcohols, amines, C₈-C₁₄ alkyl and cycloalkyl hydrocarbons and halohydrocarbons, and mixtures thereof.

Preferred solvents are selected from methoxy octadecanol, 2-(2-ethoxyethoxy)ethanol, benzyl alcohol, 2-ethylbutanol and/or 2- methylbutanol, 1-methylpropoxyethanol and/or 2-methylbutoxyethanol, linear C₁-C₅ alcohols such as methanol, ethanol, propanol, butyl diglycol ether (BDGE), butyltriglycol ether, tert-amyl alcohol, glycerol, isopropanol and mixtures thereof. Particularly preferred solvents which can be used herein are butoxy propoxy propanol, butyl diglycol ether, benzyl alcohol, butoxypropanol, propylene glycol, glycerol, ethanol, methanol, isopropanol and mixtures thereof. Other suitable solvents include propylene glycol and diethylene glycol and mixtures thereof.

**Electrolytic strength.** The electrolytic strength of the composition at a concentration of 1g/l in de-ionized water and at a temperature of 25°C in mScm⁻¹ is preferably less than 200mScm⁻¹, more preferably less than 150mScm⁻¹, even more preferably less than 100mScm⁻¹, and even less than 75mScm⁻¹, or even less than 50mScm⁻¹. The electrolytic strength can be determined by any suitable means, such as conductivity meter.

**Buffers.** The composition typically comprises buffer. Preferred buffers include mono-ethanolamine (MEA) and tri-ethanolamine (TEA). Borax may be used as a buffer, although preferably the composition is substantially free of borax, by substantially free it is typically meant no deliberately added borax is incorporated into the composition.

**Alkanolammonium cation.** Preferably, the composition comprises alkanolammonium cation, preferably mono-ethanolamine (MEA) and/or tri-ethanolamine (TEA).

**Hydrotropes.** The composition may comprise hydrotrope. A preferred hydrotrope is monopropylene glycol.

**Free water.** The composition preferably comprises less than 10wt%, or less than 5wt%, or less than 4wt% or less than 3wt% free water, or less than 2wt% free water, or less than 1wt% free water, and may even be anhydrous, typically comprising no deliberately added free water. Free water is typically measured using Karl Fischer titration. 2g of the laundry detergent composition is extracted into 50ml dry methanol at room temperature for 20 minutes and analyse 1ml of the methanol by Karl Fischer titration.

**Method of laundering fabric:** The method of laundering fabric typically comprises the step of contacting the composition to water to form a wash liquor, and laundering fabric in said wash liquor, wherein typically the wash liquor has a temperature of above 0°C to 90°C, or to 60°C, or to 40°C, or to 30°C, or to 20°C, or to 10°C, or even to 8°C. The fabric may be contacted to the water prior to, or after, or simultaneous with, contacting the laundry detergent composition with water. The composition can be used in pre-treatment applications.

The method of laundering fabric may be carried out in a top-loading or front-loading automatic washing machine, or can be used in a hand-wash laundry application. In these applications, the wash liquor formed and concentration of laundry detergent composition in the wash liquor is that of the main wash cycle. Any input of water during any optional rinsing step(s) is not included when determining the volume of the wash liquor.

### EXAMPLES

| **Ingredient** | **Amount (in wt%)** |
|---|---|
| **Anionic detersive surfactant** (such as alkyl benzene sulphonate, alkyl ethoxylated sulphate and mixtures thereof) | from 8wt% to 15wt% |
| **Non-ionic detersive surfactant** (such as alkyl ethoxylated alcohol) | from 0.5wt% to 4wt% |
| **Cationic detersive surfactant** (such as quaternary ammonium compounds) | from 0 to 4wt% |
| **Other detersive surfactant** (such as zwiterionic detersive surfactants, amphoteric surfactants and mixtures thereof) | from 0wt% to 4wt% |
| **Carboxylate polymer** (such as co-polymers of maleic acid and acrylic acid) | from 1wt% to 4wt% |
| **Polyethylene glycol polymer** (such as a polyethylene glycol polymer comprising poly vinyl acetate side chains) | from 0.5wt% to 4wt% |
| **Polyester soil release polymer** (such as Repel-o-tex and/or Texcare polymers) | from 0.1 to 2wt% |
| **Cellulosic polymer** (such as carboxymethyl cellulose, methyl cellulose and combinations thereof) | from 0.5wt% to 2wt% |
| **Other polymer** (such as amine polymers, dye transfer inhibitor polymers, hexamethylenediamine derivative polymers, and mixtures thereof) | from 0wt% to 4wt% |
| **Zeolite builder and phosphate builder** (such as zeolite 4A and/or sodium tripolyphosphate) | from 0wt% to 4wt% |
| **Other builder** (such as sodium citrate and/or citric acid) | from 0wt% to 3wt% |
| **Carbonate salt** (such as sodium carbonate and/or sodium bicarbonate) | from 15wt% to 30wt% |
| **Silicate salt** (such as sodium silicate) | from 0wt% to 10wt% |
| **Filler** (such as sodium sulphate and/or bio-fillers) | from 10wt% to 40wt% |
| **Source of available oxygen** (such as sodium percarbonate) | from 10wt% to 20wt% |
| **Bleach activator** (such as tetraacetylethylene diamine (TAED) and/or nonanoyloxybenzenesulphonate (NOBS) | from 2wt% to 8wt% |
| **Bleach catalyst** (such as oxaziridinium-based bleach catalyst and/or transition metal bleach catalyst) | from 0wt% to 0.1wt% |
| **Other bleach** (such as reducing bleach and/or pre-formed peracid) | from 0wt% to 10wt% |
| **Chelant** (such as ethylenediamine-N'N'-disuccinic acid (EDDS) and/or hydroxyethane diphosphonic acid (HEDP) | from 0.2wt% to 1wt% |
| **Photobleach** (such as zinc and/or aluminium sulphonated phthalocyanine) | from 0wt% to 0.1wt% |
| **Hueing agent** (such as direct violet 99, acid red 52, acid blue 80, direct violet 9, solvent violet 13 and any combination thereof) | from 0wt% to 1wt% |
| **Brightener** (such as brightener 15 and/or brightener 49) | from 0.1wt% to 0.4wt% |
| **Protease** (such as Savinase, Savinase Ultra, Purafect, FN3, FN4 and any combination thereof) | from 0.1wt% to 0.4wt% |
| **Amylase** (such as Termamyl, Termamyl ultra, Natalase, Optisize, Stainzyme, Stainzyme Plus and any combination thereof) | from 0.05wt% to 0.2wt% |
| **Cellulase** (such as Carezyme and/or Celluclean) | from 0.05wt% to 0.2wt% |
| **Lipase** (such as Lipex, Lipolex, Lipoclean and any combination thereof) | from 0.2 to 1wt% |
| **Other enzyme** (such as xyloglucanase, cutinase, pectate lyase, mannanase, bleaching enzyme) | from 0wt% to 2wt% |
| **Fabric softener** (such as montmorillonite clay and/or polydimethylsiloxane (PDMS) | from 0wt% to 4wt% |
| **Flocculant** (such as polyethylene oxide) | from 0wt% to 1wt% |
| **Suds suppressor** (such as silicone and/or fatty acid) | from 0wt% to 0.1wt% |
| **Perfume** (such as perfume microcapsule, spray-on perfume, starch encapsulated perfume accords, perfume loaded zeolite, and any combination thereof) | from 0.1wt% to 1wt% |
| **Aesthetics** (such as coloured soap rings and/or coloured speckles/noodles) | from 0wt% to 1wt% |
| **Miscellaneous** | balance |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A detergent composition comprising:
(a) peptidoglycan-digesting enzyme having an amino acid sequence identity of at least 80% to the amino acid SEQ ID NO: 1, 2 or 3;
(b) detersive surfactant;
and a detergent ingredient selected from the group consisting of:
(c) perfume microcapsule;
(d) a builder system that is:
(i) substantially free of phosphate builder;
(ii) substantially free of zeolite builder; and
(iii) optionally, substantially free of silicate salt;
(e) protease system comprising:
(i) protease; and
(ii) stabiliser system selected from the group consisting of: borate, polyol, 4-formylphenylboronic acid, encapsulating material, and any mixture thereof; and
(f) any mixture thereof.

2. Detergent composition according to claim 1, wherein the peptidoglycan-digesting enzyme is in encapsulated form.

3. Detergent composition according to any preceding claim, wherein the composition comprises a detergent ingredient selected from the group consisting of:
(i) starch encapsulated perfume accord;
(ii) schiff base reaction product of polyamine with perfume ketone;
(iii) cationic polymer;
(iv) fabric-softening system comprising clay and silicone;
(v) fabric-deposition aid;
(vi) alkoxylated polyamine;
(vii) mid-chain branched detersive surfactant; and
(viii) any mixture thereof.

4. Detergent composition according to any preceding claim, wherein the composition comprises bleach catalyst.

5. Detergent composition according to any preceding claim, wherein the composition comprises hueing agent.

6. Detergent composition according to any preceding claim, wherein the composition is a solid laundry detergent composition.

7. Detergent composition according to any of claims 1-6, wherein the composition is a liquid laundry detergent composition.

8. Detergent composition according to claim 7, wherein the composition has a pH of at least 7.5.

9. Detergent composition according to any preceding claim, wherein the composition is a dishwashing detergent composition.

10. Detergent composition according to any preceding claim, wherein the composition is in unit dose form.

11. Method of treating a substrate comprising the step of contacting a detergent composition according to any preceding claims with the substrate in water at a temperature of 20°C or less.

12. Use of a composition according to any of claims 1-11, for the reduction of malodour.


SEQUENCE LISTING

<110>  THE PROCTER & GAMBLE COMPANY

<120>  DETERGENT COMPOSITION COMPRISING PEPTIDOGLYCAN-DIGESTING ENZYME

<130>  CM3698FL

<160>  3

<170>  PatentIn version 3.5

<210>  1
<211>  563
<212>  PRT
<213>  Bacillus Licheniformis

<400>  1

Asp Ser Gly Lys Asn Tyr Lys Ile Ile Gly Tyr Tyr Pro Ser Trp Gly
1               5                   10                  15

Ala Tyr Gly Arg Asp Phe Gln Val Trp Asp Met Asp Val Ser Lys Val
            20                  25                  30

Ser Asn Ile Asn Tyr Ala Phe Ala Asp Ile Cys Trp Glu Gly Arg His
        35                  40                  45

Gly Asn Pro Asp Pro Thr Gly Pro Asn Pro Gln Thr Trp Ser Cys Gln
    50                  55                  60

Asp Glu Asn Gly Val Ile Asp Ala Pro Asn Gly Thr Ile Val Met Gly
65                  70                  75                  80

Asp Pro Trp Ile Asp Ala Gln Lys Ala Asn Pro Gly Asp Val Trp Asp
                85                  90                  95

Glu Pro Ile Arg Gly Asn Phe Lys Gln Leu Leu Lys Leu Lys Lys Ser
            100                 105                 110

His Pro His Leu Lys Thr Phe Ile Ser Val Gly Gly Trp Thr Trp Ser
        115                 120                 125

Asn Arg Phe Ser Asp Val Ala Ala Asp Pro Ala Ala Arg Glu Asn Phe
    130                 135                 140

Ala Ala Ser Ala Val Glu Phe Leu Arg Lys Tyr Gly Phe Asp Gly Val
145                 150                 155                 160

Asp Leu Asp Trp Glu Tyr Pro Val Ser Gly Gly Leu Pro Gly Asn Ser
                165                 170                 175

Thr Arg Pro Glu Asp Lys Arg Asn Tyr Thr Leu Leu Leu Gln Glu Val
            180                 185                 190

Arg Lys Lys Leu Asp Ala Ala Glu Ala Lys Asp Gly Lys Glu Tyr Leu
        195                 200                 205

Leu Thr Ile Ala Ser Gly Ala Ser Pro Asp Tyr Val Ser Asn Thr Glu
    210                 215                 220

Leu Asp Lys Ile Ala Gln Thr Val Asp Trp Ile Asn Ile Met Thr Tyr
225                 230                 235                 240

Asp Phe Asn Gly Gly Trp Gln Ser Ile Ser Ala His Asn Ala Pro Leu
                245                 250                 255

Phe Tyr Asp Pro Lys Ala Lys Glu Ala Gly Val Pro Asn Ala Glu Thr
            260                 265                 270

Tyr Asn Ile Glu Asn Thr Val Lys Arg Tyr Lys Glu Ala Gly Val Lys
        275                 280                 285

Gly Asp Lys Leu Val Leu Gly Thr Pro Phe Tyr Gly Arg Gly Trp Ser
    290                 295                 300

Gly Cys Glu Pro Gly Gly His Gly Glu Tyr Gln Lys Cys Gly Pro Ala
305                 310                 315                 320

Lys Glu Gly Thr Trp Glu Lys Gly Val Phe Asp Phe Ser Asp Leu Glu
                325                 330                 335

Arg Asn Tyr Val Asn Gln Asn Gly Tyr Lys Arg Tyr Trp Asn Asp Gln
            340                 345                 350

Ala Lys Val Pro Phe Leu Tyr Asn Pro Glu Asn Gly Asn Phe Ile Thr
        355                 360                 365

Tyr Asp Asp Glu Gln Ser Phe Gly His Lys Thr Asp Phe Ile Lys Ala
    370                 375                 380

Asn Gly Leu Ser Gly Ala Met Phe Trp Asp Phe Ser Gly Asp Ser Asn
385                 390                 395                 400

Arg Thr Leu Leu Asn Lys Leu Ala Ala Asp Leu Asp Phe Ala Pro Asp
                405                 410                 415

Gly Gly Asn Pro Glu Pro Pro Ser Ser Ala Pro Val Asn Val Arg Val
            420                 425                 430

Thr Gly Lys Thr Ala Thr Ser Val Ser Leu Ala Trp Asp Ala Pro Ser
        435                 440                 445

Ser Gly Ala Asn Ile Ala Glu Tyr Val Val Ser Phe Glu Asn Arg Ser
    450                 455                 460

Ile Ser Val Lys Glu Thr Ser Ala Glu Ile Gly Gly Leu Lys Pro Gly
465                 470                 475                 480

Thr Ala Tyr Ser Phe Thr Val Ser Ala Lys Asp Ala Asp Gly Lys Leu
                485                 490                 495

His Ala Gly Pro Thr Val Glu Val Thr Thr Asn Ser Asp Gln Ala Cys
            500                 505                 510

Ser Tyr Asp Glu Trp Lys Glu Thr Ser Ala Tyr Thr Gly Gly Glu Arg
        515                 520                 525

Val Ala Phe Asn Gly Lys Val Tyr Glu Ala Lys Trp Trp Thr Lys Gly
    530                 535                 540

Asp Arg Pro Asp Gln Ser Gly Glu Trp Gly Val Trp Arg Leu Ile Gly
545                 550                 555                 560

Gly Cys Glu

<210>  2
<211>  218
<212>  PRT
<213>  Aspergillus fumigatus

<400>  2

Leu Pro Ser Gln Pro Glu Ala Arg Ala Thr Thr Val Gln Gly Phe Asp
1               5                   10                  15

Ile Ser Asn His Gln Lys Ser Val Asn Phe Glu Ala Ala Lys Lys Asp
            20                  25                  30

Gly Ala Gln Phe Val Met Ile Lys Ala Thr Glu Gly Thr Thr Tyr Lys
        35                  40                  45

Asp Thr Val Phe Asn Ser His Tyr Thr Gly Ala Thr Lys Ala Gly Leu
    50                  55                  60

Leu Arg Gly Gly Tyr His Phe Ala Arg Pro Asp Lys Ser Thr Gly Ser
65                  70                  75                  80

Thr Gln Ala Lys Phe Phe Leu Lys Asn Gly Gly Gly Trp Ser Asp Asp
                85                  90                  95

Asn Arg Thr Leu Pro Gly Met Leu Asp Ile Glu Tyr Asn Pro Tyr Gly
            100                 105                 110

Ala Thr Cys Tyr Gly Leu Ser His Ser Gln Met Val Ala Trp Ile His
        115                 120                 125

Asp Phe Val Asn Glu Tyr His His Ala Thr Ser Arg Trp Pro Met Ile
    130                 135                 140

Tyr Thr Thr Ala Asp Trp Trp Asn Arg Cys Thr Gly Asn Ala Lys Gly

145                 150                 155                 160

Phe Gly Asp Lys Cys Pro Leu Val Leu Ala Ala Tyr Ser Ser Ser Pro
                165                 170                 175

Pro Lys Thr Ile Pro Gly Asp Trp Lys Thr Trp Thr Ile Trp Gln Asn
            180                 185                 190

Ser Asp Lys Tyr Lys His Gly Gly Asp Ser Asp Lys Phe Asn Gly Pro
        195                 200                 205

Met Thr Gln Leu Arg Lys Leu Ala Ser Gly
    210                 215

<210>  3
<211>  218
<212>  PRT
<213>  Aspergillus fumigatus

<400>  3

Leu Pro Ser Gln Pro Glu Ala Arg Ala Thr Thr Val Gln Gly Phe Asp
1               5                   10                  15

Ile Ser Asn His Gln Lys Ser Val Asn Phe Glu Ala Ala Lys Lys Asp
            20                  25                  30

Gly Ala Gln Phe Val Met Ile Lys Ala Thr Glu Gly Thr Thr Asp Lys
        35                  40                  45

Asp Thr Val Phe Asn Ser His Tyr Thr Gly Ala Thr Lys Ala Gly Leu
    50                  55                  60

Leu Arg Gly Gly Tyr His Phe Ala Arg Pro Asp Lys Ser Thr Gly Ser
65                  70                  75                  80

Thr Gln Ala Lys Phe Phe Leu Lys Asn Gly Gly Gly Trp Ser Asp Asp
                85                  90                  95

Asn Arg Thr Leu Pro Gly Met Leu Asp Ile Glu Tyr Asn Pro Tyr Gly
            100                 105                 110

Ala Thr Cys Tyr Gly Leu Ser His Ser Gln Met Val Ala Trp Ile His
        115                 120                 125

Asp Phe Val Asn Glu Tyr His His Ala Thr Ser Arg Trp Pro Met Ile
    130                 135                 140

Tyr Thr Thr Ala Asp Trp Trp Asn Arg Cys Thr Gly Asn Ala Lys Gly
145                 150                 155                 160

Phe Gly Asp Lys Cys Pro Leu Val Leu Ala Ala Tyr Ser Ser Ser Pro
                165                 170                 175

Pro Lys Thr Ile Pro Gly Asp Trp Lys Thr Trp Thr Ile Trp Gln Asn
            180                 185                 190

Ser Asp Lys Tyr Lys His Gly Gly Asp Ser Asp Lys Phe Asn Gly Pro
        195                 200                 205

Met Thr Gln Leu Arg Lys Leu Ala Ser Gly
    210                 215